# EUROPÄISCHE PATENTSCHRIFT

(11) **EP 4 021 378 B1**
(45) Veröffentlichungstag und Bekanntmachung des Hinweises auf die Patenterteilung: **11.09.2024**
(21) Anmeldenummer: 20747380.2
(22) Anmeldetag: 27.07.2020
(51) Int. Cl.: A61K 8/06, A61K 8/36, A61K 8/73, A61Q 19/00

(54) **ACRYLAT- UND SILIKONFREIE KOSMETISCHE O/W-EMULSION**
ACRYLATE AND SILICON-FREE COSMETIC O/W EMULSION
ÉMULSION H/E COSMÉTIQUE EXEMPTE D'ACRYLATE ET DE SILICONE

(30) Priorität: 28.08.2019 DE 102019212891
(43) Veröffentlichungstag der Anmeldung: 06.07.2022
(73) Patentinhaber: Beiersdorf AG, 22529 Hamburg (DE)
(72) Erfinder: FRANCK, Kerstin, 25451 Quickborn (DE); SKUBSCH, Kerstin, 25497 Prisdorf (DE)
(74) Vertreter: Beiersdorf AG
(86) Internationale Anmeldenummer: PCT/EP2020/071104
(87) Internationale Veröffentlichungsnummer: WO 2021/037456

(56) Entgegenhaltungen:
- EP-A1- 4 021 380
- EP-A1- 4 021 381
- EP-A1- 4 021 382
- EP-A2- 1 352 641
- WO-A1-2008/003685
- WO-A2-2007/017196
- US-A1- 2016 074 310
- US-A1- 2019 209 446

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Zur Stabilisierung und zur Verdickung von O/W-Emulsionen werden zumeist Acrylat-basiete Polymere in diese Formulierungen eingearbeitet. Acrylat-basierte Polymere sind Polymere, welche aus Homo- oder Copolymerisation mit Acryl- und/oder Methacrylsäure erhalten werden. Beispiele hierfür sind u.a. Carbomer oder Acrylate Copolymer.

Der Stand der Technik kennt unter anderem die Dokumente DE 10148825 A1, DE 19938756 A1 und DE 29924371 U1, welche pflegende O/W-Emulsionen mit Acrylat-basierten Polymeren offenbaren. Jedoch ist der Einsatz dieser Acrylat-basierten Polymere zunehmend in der Kritik, da deren biologische Abbaubarkeit nicht vollständig geklärt ist.

Weiterhin kennt der Stand der Technik das Dokument WO2007/017169 A2, welches Öl in Wasser Emulsionen mit mindestens einem hydrophob modifizierten Polysaccharid und mindestens einem Copolymer basierend auf einer Acrylamidoalkansäure enthalten. Unter anderem umfassen die offenbarten Emulsionen Hydroxypropylstärkephosphat.

Ein weiterer oft verwendeter Bestandteil von O/W-Emulsionen zur Auftragung auf die Haut sind Silikonöle. Bei Auftragen einer O/W-Emulsion fühlt sich die Haut geschmeidig an. Zudem haben Silikonöle einen weichzeichnenden Effekt und wirken als optischer Faltenfiller. Silikone sind per se für die Haut nicht schädlich. Als hautfremde Stoffe lösen sie keine Allergien oder Reizreaktionen aus. Einmal abgewaschen ist die Haut wieder wie vorher.

Kritisch ist jedoch ebenfalls der Umstand dass die Umweltverträglichkeit von Silikonölen noch nicht abschließend beurteilt wurde bzw. sich die Substanzen in der Natur nur schwer abbauen. Folglich gilt es den Einsatz von Silikonölen in kosmetischen Zubereitungen zur Auftragung auf die Haut zu reduzieren.

Problematisch ist jedoch der Umstand, dass O/W-Emulsionen die keine acrylathaltigen Polymere und bei denen auf den Einsatz von Silikonölen verzichtet wird oftmals dazu tendieren beim Verteilen auf der Haut weiße Rückstände zu hinterlassen.

Aufgabe der vorliegenden Erfindung war es daher eine acrylat- und silikonfreie kosmetische O/W-Emulsion bereitzustellen, welche die Nachteile des Standes der Technik nicht aufweist. Insbesondere war es Aufgabe eine derartige O/W-Emulsion bereitzustellen, welche beim Auftragen schnell einzieht und dabei geringere Mengen an Rückständen aufweist.

Überraschend gelöst wird/werden die Aufgaben durch eine acrylat- und silikonfreie kosmetische O/W-Emulsion enthaltend
a) von 0,1 bis 6 Gew.-% mindestens eine Fettsäure aufweisend 12 bis 22 Kohlenstoffatome, und
b) von 0,1 bis 5 Gew.-% Hydroxypropylstärkephosphat;
jeweils bezogen auf das Gesamtgewicht der Emulsion,
dadurch gekennzeichnet, dass
- die O/W-Emulsion kein Mineralöl enthält,
- Palmitinsäure und Stearinsäure enthalten sind, und
- neben Palmitinsäure und Stearinsäure weitere Fettsäuren enthalten sind, wobei das Gewichtsverhältnis aus dem Gesamtanteil der Palmitinsäure und Stearinsäure zu dem Gesamtanteil der weiteren Fettsäuren von 8:1 bis 99:1 beträgt.

Gegenstand der Erfindung ist auch die Verwendung eine Mischung aus
a) mindestens eine Fettsäure aufweisend 12 bis 22 Kohlenstoffatome, und
b) Hydroxypropylstärkephosphat,
in einer acrylat- und silikonfreien kosmetische O/W-Emulsion zur Reduzierung von weißen Rückständen beim Auftragen der Emulsion auf die Haut, dadurch gekennzeichnet, dass
- die O/W-Emulsion kein Mineralöl enthält,
- Palmitinsäure und Stearinsäure enthalten sind, und
- neben Palmitinsäure und Stearinsäure weitere Fettsäuren enthalten sind, wobei das Gewichtsverhältnis aus dem Gesamtanteil der Palmitinsäure und Stearinsäure zu dem Gesamtanteil der weiteren Fettsäuren von 8:1 bis 99:1 beträgt.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen O/W-Emulsion. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der kosmetischen O/W-Emulsion angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer sowohl auf die erfindungsgemäße Zubereitung sowie die erfindungsgemäße Verwendung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Die erfindungsgemäße O/W-Emulsion ist acrylatfrei. Acrylatfrei bedeutet im Sinne der vorliegenden Erfindung, dass der Gesamtanteil an Acrylat-basierten Polymeren weniger als 0,05 Gew.-% und insbesondere bevorzugt 0 Gew-% beträgt, wobei sich die Angaben auf das Gesamtgewicht der Emulsion beziehen. Erfindungsgemäß werden unter Acrylat-basierten Polymeren alle Polymere verstanden, welche aus einer Homo- oder Copolymerisation mit Acryl- und/oder Methacrylsäure gewonnen werden.

Im Sinne der vorliegenden Offenbarung bedeutet silikonfrei, dass keine Moleküle aufweisend Dialkylsiloxan-Einheiten enthalten sind.

Erfindungsgemäß umfasst die kosmetische O/W-Emulsion mindestens eine Fettsäure aufweisend 12 bis 22 Kohlenstoffatome. Bevorzugte Fettsäuren sind gewählt aus der Gruppe Palmitinsäure, Stearinsäure, Myristinsäure, Arachidinsäure sowie Ölsäure.

Es sind mindestens Palmitinsäure und Stearinsäure enthalten.

Der Gesamtanteil der Fettsäuren aufweisend 12 bis 22 Kohlenstoffatome in der erfindungsgemäßen Emulsion beträgt von 0,1 bis 6 Gew.-%, bezogen auf das Gesamtgewicht der Emulsion. Es ist bevorzugt, wenn der Gesamtanteil der Fettsäuren aufweisend 12 bis 22 Kohlenstoffatome von 0,2 bis 5 Gew.-% und insbesondere bevorzugt von 0,5 bis 3 Gew.-% beträgt, jeweils bezogen auf das Gesamtgewicht der Emulsion.

Dabei ist es weiterhin vorteilhaft, wenn mindestens Palmitinsäure enthalten ist und der Anteil von Palmitinsäure bevorzugt von 0,4 bis 2,2 Gew.-%, bezogen auf das Gesamtgewicht der Emulsion, beträgt.

Es ist weiterhin vorteilhaft, wenn mindestens Stearinsäure enthalten ist und der Anteil von Stearinsäure bevorzugt von 0,4 bis 2,2 Gew.-%, bezogen auf das Gesamtgewicht der Emulsion, beträgt.

Die Erfindung ist ferner dadurch gekennzeichnet, dass neben Palmitinsäure und Stearinsäure weitere Fettsäuren enthalten sind, wobei das Gewichtsverhältnis aus dem Gesamtanteil der Palmitinsäure und Stearinsäure zu dem Gesamtanteil der weiteren Fettsäuren von 8:1 bis 99:1 beträgt.

Weiterhin ist es vorteilhaft im Sinne der vorliegenden Erfindung, wenn der Anteil von Hydroxypropylstärkephosphat von 0,2 Gew.-% bis 3,8 Gew.-%, bevorzugt von 0,3 Gew.-% bis 3,5 Gew.-% und insbesondere bevorzugt von 0,4 Gew.-% bis 3 Gew.-%, bezogen auf das Gesamtgewicht der Emulsion, beträgt.

Generell ist Hydroxypropylstärkephosphat ein Veresterungsprodukt basierend auf Stärke. Erfindungsgemäß ist es möglich Hydroxypropylstärkephosphat basierend auf verschiedenen Stärken einzusetzen. Dem Fachmann sind unter anderem Weizen- oder Kartoffelstärke bekannt.

Vorteilhafte Ausführungsformen der vorliegenden Erfindung sind dadurch gekennzeichnet, dass das eingesetzte Hydroxypropylstärkephosphat ein Veresterungsprodukt basierend auf Maisstärke ist. Innerhalb dieser Ausführungsformen ist es weiter bevorzugt, wenn der Anteil des Hydroxypropylstärkephosphat, welches ein Veresterungsprodukt basierend auf Maisstärke ist, von 0,2 Gew.-% bis 3,8 Gew.-%, bevorzugt von 0,3 Gew.-% bis 3,5 Gew.-% und insbesondere bevorzugt von 0,4 Gew.-% bis 3 Gew.-%,, bezogen auf das Gesamtgewicht der Emulsion, beträgt.

Ein erfindungsgemäßes Hydroxypropylstärkephosphat basierend auf Maisstärke ist unter dem Handelsnamen C*HiForm A 12747 von Cargill oder Structure^{®} XL von Akzo Nobel Specialty Chemicals zu beziehen.

Es ist ebenfalls erfindungsgemäß von Vorteil, wenn Glyceryl Stearate enthalten ist. Ist Glyceryl Stearate enthalten, so ist es ebenfalls vorteilhaft, wenn der Gesamtanteil von Glyceryl Stearate von 0,1 Gew.-% bis 5,0 Gew.-%, bevorzugt 0,5 Gew.-% bis 3,2 Gew.-% und insbesondere bevorzugt von 1,0 Gew.-% bis 2,5 Gew.-%, beträgt, wobei sich die Angaben auf das Gesamtgewicht der Emulsion beziehen.

Ferner ist es vorteilhaft, wenn Glyceryl Stearate Citrate enthalten ist. Ist Glyceryl Stearate Citrate enthalten, so ist es ebenfalls vorteilhaft, wenn der Gesamtanteil von Glyceryl Stearate Citrate von 0,1 Gew.-% bis 5,0 Gew.-%, bevorzugt 0,5 Gew.-% bis 3,2 Gew.-% und insbesondere bevorzugt von 0,8 Gew.-% bis 2,5 Gew.-%, beträgt, wobei sich die Angaben auf das Gesamtgewicht der Emulsion beziehen.

Ferner ist es vorteilhaft, wenn Polyglyceryl-3 Diisostearate enthalten ist. Ist Polyglyceryl-3 Diisostearate enthalten, so ist es ebenfalls vorteilhaft, wenn der Gesamtanteil von Polyglyceryl-3 Diisostearate von 0,1 Gew.-% bis 3,1 Gew.-%, bevorzugt 0,15 Gew.-% bis 1,5 Gew.-% und insbesondere bevorzugt von 0,2 Gew.-% bis 1',0 Gew.-%, beträgt, wobei sich die Angaben auf das Gesamtgewicht der Emulsion beziehen.

Ferner ist es vorteilhaft, wenn Polyglyceryl-2 Caprate enthalten ist. Ist Polyglyceryl-2 Caprate enthalten, so ist es ebenfalls vorteilhaft, wenn der Gesamtanteil von Polyglyceryl-2 Caprate von 0,1 Gew.-% bis 0,5 Gew.-%, bevorzugt 0,15 Gew.-% bis 0,4 Gew.-% beträgt, wobei sich die Angaben auf das Gesamtgewicht der Emulsion beziehen.

Weiterhin wurde überraschend gefunden, dass sich insbesondere Emulsionen mit der erfindungsgemäßen Kombination stabilisieren lassen, welche dadurch gekennzeichnet sind, dass der Anteil der Ölphase der Emulsion von mehr als 2 Gew.-% bis 30 Gew.-%, bevorzugt von mehr als 5 Gew.-% bis 20 Gew.-% und insbesondere bevorzugt von mehr als 8 Gew.-% bis 15,5 Gew.-%, bezogen auf das Gesamtgewicht der Emulsion, beträgt, wobei Tenside und Emulgatoren definitionsgemäß nicht zur Ölphase zählen, es sei denn sie sind in der vorliegenden Offenbarung explizit der Ölphase zugeordnet. Somit lassen sich überraschender Weise Emulsionen mit einem großen Ölphasen-Anteil stabilisieren.

Erfindungsgemäß zählen Emulgatoren und Tenside nicht zur Ölphase. Das heißt, dass unter anderem Glyceryl Stearate, Glyceryl Stearate Citrate und Polyglyceryl-3 Diisostearate nicht zur Ölphase zählen.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Es hat sich weiterhin überraschend gezeigt, dass die Konsistenz und somit die Zähflüssigkeit der erfindungsgemäßen O/W-Emulsion weiter gesteigert werden konnte, indem der Emulsion ein oder mehrere Fettalkohole mit 14 bis 22 Kohlenstoffatomen zugesetzt werden. Der Gesamtanteil dieser Fettalkohole mit 14 bis 22 Kohlenstoffatomen beträgt vorteilhaft von 0,1 Gew.-% bis 8,0 Gew.-%, bevorzugt 0,5 Gew.-% bis 7,0 Gew.-% und insbesondere bevorzugt von 1 Gew.-% bis 6,0 Gew.-%,bezogen auf das Gesamtgewicht der Emulsion. Fettalkohole mit 14 bis 22 Kohlenstoffatomen zählen zur Ölphase.

Insbesondere bevorzugt zu wählende Fettalkohole sind Myristylalkohol, Cetylalkohol und/oder Stearylalkohol, wobei diese bevorzugt in einem Gesamtanteil von 0,1 Gew.-% bis 8,0 Gew.-%, bevorzugt 2 Gew.-% bis 7,0 Gew.-% und insbesondere bevorzugt von 3 Gew.-% bis 6,0 Gew.-%, bezogen auf das Gesamtgewicht der Emulsion, eingesetzt werden. Die Mischung aus Cetylalkohol und Stearylalkohol ist unter dem INCI Namen Cetearylalkohol bekannt.

Vorteilhafte Ausführungsformen der vorliegenden Erfindung sind weiterhin dadurch gekennzeichnete, dass die O/W-Emulsion weitere Polysaccharid haltige Polymere enthält.

Andere vorteilhafte Ausführungsformen der vorliegenden Erfindung sind ferner dadurch gekennzeichnet, dass diese neben Hydoxypropylstärkephosphat keine weiteren Stärken und Stärkederivate enthält. Es war insbesondere überraschend, dass auf den weiteren Einsatz von weiteren Polymeren verzichtet werden konnte.

Andere vorteilhafte Ausführungsformen der vorliegenden Erfindung sind ferner dadurch gekennzeichnet, dass diese keine Cellulosen oder Cellulosederivate enthält.

Andere vorteilhafte Ausführungsformen der vorliegenden Erfindung sind ferner dadurch gekennzeichnet, dass diese keine weiteren Polysaccharid-haltige Polymere enthält.

Die erfindungsgemäße O/W-Emulsion enthält weiterhin vorteilhaft ein oder mehrere Lipidkomponenten, wie Wachse und Öle auf Basis von Kohlenwasserstoffen, gesättigte, ungesättige oder gehärtete Triglyceride, Dialkylether mit 12 bis 24 Kohlenstoffatomen und/oder die Ester aus einwertigen Alkoholen und Monocarbonsäuren, welche mindestens 10 Kohlenstoffatome aufweisen.

Wie bereits ausgeführt, ist es erfindungsgemäß, dass die O/W-Emulsion der vorliegenden Erfindung kein Mineralöl enthält.

Ferner ist es vorteilhaft, wenn keine verzweigten und/oder unverzweigten Kohlenwasserstoffe enthalten sind. Beispiele derartiger Kohlenwasserstoffe sind Paraffinum Liquidum, Isododecan, Isohexadecan, Isoeicosane, Squalan und Cera Microcristallina.

Die erfindungsgemäße O/W-Emulsion enthält vorteilhaft ein oder mehrere Triglycerinester, gewählt aus der Gruppe der synthetischen, halbsynthetischen und natürlichen Triglycerinester. Es ist insbesondere bevorzugt, wenn die Ölphase unter den Trigkycerinestern ausschließlich natürliche Triglycerinester enthält.

Vorteilhaft enthaltene natürliche Öle sind gewählt aus der Gruppe Persea Gratissima Oil, Orbignya Oleifera Seed Oil, Argania Spinosa Kernel Oil, Prunus Armeniaca Kernel Oil, Simmondsia Chinensis Seed Oil, Cocos Nucifera Oil, Silybum Marianum Seed Oil, Oenothera Biennis Oil, Olea Europaea Fruit Oil, Helianthus Annuus Seed Oil, Vitis Vinifera Seed Oil, Cannabis Sativa Seed Oil, Olus Oil, Gossypium Herbaceum Seed Oil, Arctium Lappa Seed Oil, Macadamia Ternifolia Seed Oil, Zea Mays Germ Oil, Prunus Amygdalus Dulcis Oil, Ricinus Communis Seed Oil, Vegetable Oil und Glycine Soja Oil.

Enthält die O/W-Emulsion ein oder mehrere der vorstehend genannten natürlichen Öle, so beträgt der Anteil dieser natürlichen Öle bevorzugt von 0,1 Gew.-% bis 5,0 Gew.-%, bezogen auf das Gesamtgewicht der O/W-Emulsion.

Es ist ebenfalls vorteilhaft, wenn ein Öl gewählt aus der Gruppe Coco-Caprylate, Dicaprylyl Ether, Cocoglycerides, Coco-Caprylate/Caprate, Decyl Oleate, Caprylic/ CapricTriglyceride, Ethylhexyl Cocoate, Octyldodecanol, Squalane, Triisostearin, Shea Butter Ethyl Esters, Ethylhexyl Cocoate, Decyl Cocoate, Isoamyl Cocoate, Caprylyl Caprylate/ Caprate, Triheptanoin, Hexyldecyl Sterate und Isoamyl Laurate enthalten ist.

Zu den weiteren bevorzugten Triglycerinestern zählen unter anderen gehärtete Triglyceridfette, wie hydriertes Palmöl, hydriertes Kokosöl, hydriertes Rizinusöl oder Tristearin. Insbesondere ist der Einsatz von hydriertem Kokosöl (Hydrogenated Coco-Glycerides) bevorzugt, wobei der Anteil von hydriertem Kokosöl bevorzugt von 0,5 Gew.-% bis 3 Gew.-%, bezogen auf das Gesamtgewicht der Emulsion, beträgt.

Ferner ist es vorteilhaft im Sinne der vorliegenden Erfindung, wenn die O/W-Emulsion der vorliegenden Erfindung ein oder mehrere Dialkylether mit 12 bis 24 Kohlenstoffatomen enthält, wobei bevorzugt Dicaprylylether enthalten ist. Enthält die O/W-Emulsion Dicaprylylether, so beträgt der Anteil von Dicaprylylether bevorzugt von 0,1 Gew.-% bis 3 Gew.-%, bezogen auf das Gesamtgewicht der O/W-Emulsion.

Es ist weiterhin vorteilhaft im Sinne der vorliegenden Erfindung, wenn die O/W-Emulsion Cetylpalmitat enthält. Ist Cetylpalmitat enthalten, so beträgt der Anteil von Cetylpalmitat bevorzugt von 0,1 bis 5 Gew.-%, bezogen auf das Gesamtgewicht der Emulsion.

Es ist weiterhin vorteilhaft im Sinne der vorliegenden Erfindung, wenn die O/W-Emulsion Octyldodecanol enthält. Ist Octyldodecanol enthalten, so beträgt der Anteil von Octyldodecanol bevorzugt von 0,1 bis 2 Gew.-%, bezogen auf das Gesamtgewicht der Emulsion.

Ferner ist es insbesondere vorteilhaft, wenn die Emulsion dadurch gekennzeichnet ist, dass sie Coco-Caprylate und/oder Coco-Caprylate/Caprate enthält, wobei der Gesamtanteil von Coco-Caprylate und/oder Coco-Caprylate/Caprate bevorzugt von 0,5 bis 7 Gew.-% und insbesondere bevorzugt von 2 bis 6 Gew.-% bezogen auf das Gesamtgewicht der Emulsion beträgt.

Ferner ist es insbesondere vorteilhaft, wenn die Emulsion dadurch gekennzeichnet ist, dass sie Triisostearin und/oder Tristearin enthält, wobei der Gesamtanteil von Triisostearin und/oder Tristearin bevorzugt von 0,5 bis 7 Gew.-% und insbesondere bevorzugt von 1,3 bis 3,5 Gew.-% bezogen auf das Gesamtgewicht der Emulsion beträgt.

Ein weiterer insbesondere vorteilhafter Bestandteil der der Emulsion ist weiterhin Butyrospermum Parkii Butter, welche bevorzugt in ein Anteil von 0,3 bis 2 Gew.-% und insbesondere bevorzugt von 0,5 bis 1,5 Gew.-% bezogen auf das Gesamtgewicht der Emulsion enthalten ist.

Es ist darüber hinaus erfindungsgemäß von Vorteil, wenn die O/W-Emulsion Propylenglycol, Butylenglycol, 2-Methylpropan-1,3-diol, 1,2-Pentandiol, 1,2-Hexandiol, 1,2-Octandiol Glycerylcaprylate und/oder 1,2-Decandiol enthält.

Weiterhin ist es ebenfalls vorteilhaft im Sinne der vorliegenden Erfindung, wenn die O/W-Emulsion Phenoxyethanol, Dehydracetsäure, Benzyl Alkohol und/oder Ethylhexylglycerin enthält.

Enthält die O/W-Emulsion Benzylalkohol so ist es bevorzugt, wenn der Anteil an Benzylalkohol von 0,1 Gew.-% bis 0,5 Gew.-% beträgt, bezogen auf das Gesamtgewicht der O/W-Emulsion.

Enthält die O/W-Emulsion Phenoxyethanol, so ist es bevorzugt, wenn der Gesamtanteil an Phenoxyethanol von 0,1 Gew.-% bis 1,2 Gew.-% beträgt, bezogen auf das Gesamtgewicht der O/W-Emulsion.

Enthält die O/W-Emulsion Ethylhexylglycerin, so ist es bevorzugt, wenn der Anteil an Ethylhexylglycerin von 0,1 Gew.-% bis 1,0 Gew.-% beträgt, bezogen auf das Gesamtgewicht der O/W-Emulsion.

Enthält die O/W-Emulsion 1,2-Octandiol, so ist es bevorzugt, wenn der Anteil an 1,2-Octandiol von 0,1 Gew.-% bis 1,0 Gew.-% beträgt, bezogen auf das Gesamtgewicht der O/W-Emulsion.

Darüber hinaus ist es vorteilhaft, wenn Ausführungsformer der Erfindung dadurch gekennzeichnet sind, dass diese Ethanol enthalten. Ist Ethanol in der O/W-Emulsion enthalten, so beträgt der Anteil von Ethanol bevorzugt von 0,5 Gew.-% bis 10 Gew.-%, bezogen auf das Gesamtgewicht der O/W-Emulsion.

Ferner ist die erfindungsgemäße O/W-Emulsion bevorzugt dadurch gekennzeichnet, dass diese Glycerin in einem Anteil von 0,5 Gew.-% bis 15% Gew.-% , bezogen auf das Gesamtgewicht der O/W-Emulsion, enthält.

Weiterhin, ist es bevorzugt, wenn die O/W-Emulsion weitere Tenside und/oder Emulgatoren nur in einem Maximalanteil von 2 Gew.-%, bevorzugt in einem Maximalanteil von 1,5 Gew.-% enthält, wobei sich die Angaben auf das Gesamtgewicht der Emulsion beziehen.

Nicht zuletzt sind erfindungsgemäß vorteilhafte Ausführungsformen dadurch gekennzeichnet, dass die O/W-Emulsion einen oder mehrere Wirkstoffe gewählt aus der Gruppe der Verbindungen Glycyrrhetinsäure, Harnstoff, Arctiin, Folsäure, Coenzym Q10 (Ubiquinon), alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, Vitamin C, Vitamin C phosphate, Vitamin C palmitate, Niacinamide, Vitamin A palmitate, Panthenol, Licochalcon A, Rucinol, N-[(2,4-Dihydroxyphenyl)thiazol-2-yl]isobutyramide, Honociol und Magnolol (auch als Bestandteil von Magnolia-Extrakten), Hyaluronsäure und/oder Silymarin (Mariendistelextrakt) enthält.

Ferner sind erfindungsgemäß vorteilhafte O/W-Emulsionen dadurch gekennzeichnet, dass diese Wasser in einem Anteil von 60 Gew.-% bis 95 Gew.-% und bevorzugt von 70 Gew.-% bis 90 Gew.-%, bezogen auf das Gesamtgewicht der Emulsion enthalten.

Weiterhin weisen erfindungsgemäß vorteilhafte O/W-Emulsionen 24 h nach Herstellung eine Viskosität von 8000 mPa·s bis 20000 mPa·s auf. Wird in dieser Offenbarung von Viskosität gesprochen, so beziehen sich alle Werte auf eine Messung bei 25°C im 150 ml Rollrandglas mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messystems wird die Viskosität berechnet. Als Messkörper wird der Messkörper Nr.2 (Artikelnr. 200 0192), Drehzahl Bereich 62,5 min⁻¹, verwendet. Alle Messungen zur Viskosität erfolgen immer 24h nach Herstellung der O/W-Emulsion.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgende Tabelle zeigt mit Vgl.1 und Vgl.2 zwei nicht erfindungsgemäße Emulsionen, während Bsp.1 eine erfindungsgemäße Emulsion darstellt. Die Eigenschaften von Vgl.1 und Vgl.2 wurden zu Bsp.1 verglichen. Ein Expertenpanel hat beim Auftragen der Emulsionen auf die Haut festgestellt, dass Vgl.1 und Vgl.2 signifikante Mengen an weißen Rückständen beim Auftragen auf dem Unterarm hinterlassen. Hingegen zeigt Bsp.1 fast keine weißen Rückstände.

| **Inhaltstoff** | **Vgl.1** | **Vgl.2** | **Bsp.1** |
|---|---|---|---|
| Fettsäuremischung bestehend aus 47% Stearinsäure, 48,5% Palmitinsäure, 2,5% Myristinsäure, 1,5% Arachidinsäure and 0,5% Ölsäure. | 3 | 3 | 3 |
| Glyceryl Stearate | 1,2 | 1,2 | 1,2 |
| Polyglyceryl-3 Diisostearate | 0,7 | 0,7 | 0,7 |
| Vegetable Oil | 1 | 1 | 1 |
| Octyldodecanol | 0,5 | 0,5 | 0,5 |
| Triisostearin | 1,5 | 1,5 | 1,5 |
| Myristyl Alcohol | 5 | 5 | 5 |
| Myristyl Myristate | 2 | 2 | 2 |
| Hydrogenated Coco-Glycerides | 1,2 | 1,2 | 1,2 |
| Phenoxyethanol | 0,35 | 0,35 | 0,35 |
| Ethylhexylglycerin | 0,2 | 0,2 | 0,2 |
| Parfum | 0,1 | 0,1 | 0,1 |
| Sodium Hydroxide | q.s. | q.s. | q.s. |
| Hydroxypropyl Starch Phosphate | | | 2 |
| Gellan Gum | 0,1 | | |
| Kappa Carrageen | | 0,4 | |
| Glycerin | 3 | 3 | 3 |
| Ethanol | 3 | 3 | 3 |
| Aqua | ad 100 | ad 100 | ad 100 |
| Weisseln beim Verteilen auf dem Unterarm (jeweils 75 µl) | stark | stark | kaum |

Die weiteren Beispiele sollen die Erfindung weiter verdeutlichen ohne sie einzuschränken.

| **Inhaltstoff** | **Bsp. 2** | **Bsp. 3** | **Bsp. 4** | **Bsp. 5** | **Bsp. 6** | **Bsp. 7** |
|---|---|---|---|---|---|---|
| Fettsäuremischung bestehend aus 47% Stearinsäure, 48,5% Palmitinsäure, 2,5% Myristinsäure, 1,5% Arachidinsäure and 0,5% Ölsäure. | 1,5 | 4 | 1 | 2 | 0,8 | 0,5 |
| Glyceryl Stearate | 1,5 | | | 2 | 1 | 1,2 |
| Glyceryl Stearate Citrate | 1,5 | 2 | 1 | | 0,8 | 0,6 |
| Polyglyceryl-3 Diisostearate + Aqua | 0,4 | | 3 | | 0,5 | 0,2 |
| Vegetable Oil | 0,1 | | | | 0,6 | 0,8 |
| Prunus Amygdalus Dulcis Oil | 0,2 | 1 | | | | 0,3 |
| Octyldodecanol | | | 0,2 | 1 | 0,4 | |
| Coco-Caprylate | | 1 | 0,2 | | | |
| Coco-Caprylate/Caprate | 0,2 | | | 1 | | |
| Caprylic/Capric Triglyceride | 0,2 | | 0,5 | | | |
| Dicaprylyl Ether | | 0,2 | | | | |
| Triisostearin | | | | 0,2 | 1,2 | |
| Tristearin | | | 0,5 | 0,2 | | 1,2 |
| Myristyl Alcohol | | 6 | | | 2 | 3 |
| Cetyl Alcohol | | | 3 | | 2 | |
| Cetearyl Alcohol | 3 | | | 4 | | |
| Myristyl Myristate | 1,5 | 1 | | 0,5 | 1,8 | 3 |
| Hydrogenated Coco-Glycerides | | | | 2 | 1 | 0,1 |
| Cetyl Palmitate | 1 | 2 | 1,5 | | | |
| Butyrospermum Parkii Butter | 1 | | 1,5 | 5 | | |
| Phenoxyethanol | 0,9 | 0,9 | 0,9 | 0,9 | | |
| Caprylyl Glycol | | | | 0,1 | 0,25 | 0,33 |
| Ethylhexylglycerin | | | 0,1 | | 0,25 | 0,4 |
| Polyglyceryl-2 Caprate | | 0,5 | | | | |
| Parfum | 0,2 | 0,4 | 0,25 | 0,4 | 0,2 | 0,15 |
| Sodium Hydroxide | q.s. | q.s. | q.s. | q.s. | q.s. | q.s. |
| Hydroxypropyl Starch Phosphate | 0,75 | 0,2 | 3 | 3 | 1,5 | 0,8 |
| Glycerin | 5 | 8 | 7 | 12 | 4 | 5 |
| Ethanol | 4 | 2 | 2 | 4 | 4 | 3 |
| Aqua | ad 100 | ad 100 | ad 100 | ad 100 | ad 100 | ad 100 |

| **Inhaltstoff** | **Bsp. 8** | **Bsp. 9** | **Bsp. 10** |
|---|---|---|---|
| Fettsäuremischung bestehend aus 47% Stearinsäure, 48,5% Palmitinsäure, 2,5% Myristinsäure, 1,5% Arachidinsäure and 0,5% Ölsäure. | 0,2 | 2 | 1 |
| Glyceryl Stearate | 3 | 0,5 | 0,8 |
| Glyceryl Stearate Citrate | 2,5 | | 1 |
| Polyglyceryl-3 Diisostearate + Aqua | | 1 | 0,8 |
| Vegetable Oil | | | 0,4 |
| Prunus Amygdalus Dulcis Oil | 0,7 | | 0,5 |
| Coco-Caprylate | | 0,5 | |
| Coco-Caprylate/Caprate | 0,5 | | |
| Caprylic/Capric Triglyceride | | 1 | |
| Dicaprylyl Ether | | 1 | |
| Triisostearin | | 2 | |
| Tristearin | 2,5 | | 2 |
| Myristyl Alcohol | | | |
| Cetyl Alcohol | | 4 | 3,5 |
| Cetearyl Alcohol | 3,5 | | |
| Myristyl Myristate | 0,2 | | 2,5 |
| Hydrogenated Coco-Glycerides | | 0,5 | 0,5 |
| Cetyl Palmitate | 3 | 0,5 | |
| Butyrospermum Parkii Butter | | 1 | |
| Caprylyl Glycol | 0,33 | 0,25 | 0,1 |
| Ethylhexylqlycerin | 0,6 | 0,25 | 0,4 |
| Polyglyceryl-2 Caprate | | 0,25 | 0,2 |
| Parfum | 0,4 | 0,25 | 0,3 |
| Aqua + Sodium Hydroxide | 0,1 | 0,02 | 0,05 |
| Hydroxypropyl Starch Phosphate | 2 | 1 | 2,5 |
| Glycerin | 6 | 10 | 6 |
| Ethanol | 4,5 | 2 | 5 |
| Aqua | ad 100 | ad 100 | ad 100 |

## Patentansprüche

1. Acrylat- und silikonfreie kosmetische O/W-Emulsion enthaltend
a) von 0,1 bis 6 Gew.-% mindestens eine Fettsäure aufweisend 12 bis 22 Kohlenstoffatome, und
b) von 0,1 bis 5 Gew.-% Hydroxypropylstärkephosphat;
jeweils bezogen auf das Gesamtgewicht der Emulsion,
**dadurch gekennzeichnet, dass**
- die O/W-Emulsion kein Mineralöl enthält,
- Palmitinsäure und Stearinsäure enthalten sind, und
- neben Palmitinsäure und Stearinsäure weitere Fettsäuren enthalten sind, wobei das Gewichtsverhältnis aus dem Gesamtanteil der Palmitinsäure und Stearinsäure zu dem Gesamtanteil der weiteren Fettsäuren von 8:1 bis 99:1 beträgt.

2. Verwendung eine Mischung aus
a) mindestens eine Fettsäure aufweisend 12 bis 22 Kohlenstoffatome, und
b) Hydroxypropylstärkephosphat,
in einer acrylat- und silikonfreien kosmetische O/W-Emulsion zur Reduzierung von weißen Rückständen beim Auftragen der Emulsion auf die Haut, **dadurch gekennzeichnet, dass**
- die O/W-Emulsion kein Mineralöl enthält,
- Palmitinsäure und Stearinsäure enthalten sind, und
- neben Palmitinsäure und Stearinsäure weitere Fettsäuren enthalten sind, wobei das Gewichtsverhältnis aus dem Gesamtanteil der Palmitinsäure und Stearinsäure zu dem Gesamtanteil der weiteren Fettsäuren von 8:1 bis 99:1 beträgt.

3. Emulsion nach Anspruch 1 oder Verwendung nach Anspruch 2 **dadurch gekennzeichnet, dass** die Fettsäuren gewählt sind aus der Gruppe Palmitinsäure, Stearinsäure, Myristinsäure, Arachidinsäure sowie Ölsäure.

4. Emulsion oder Verwendung nach einem der vorhergehenden Ansprüche **dadurch gekennzeichnet, dass** der Gesamtanteil der Fettsäuren aufweisend 12 bis 22 Kohlenstoffatome von 0,2 bis 5 Gew.-% und bevorzugt von 0,5 bis 3 Gew.-% beträgt, jeweils bezogen auf das Gesamtgewicht der Emulsion.

5. Emulsion oder Verwendung nach einem der vorhergehenden Ansprüche **dadurch gekennzeichnet, dass** der Anteil von Palmitinsäure von 0,4 bis 2,2 Gew.-%, bezogen auf das Gesamtgewicht der Emulsion, beträgt.

6. Emulsion oder Verwendung nach einem der vorhergehenden Ansprüche **dadurch gekennzeichnet, dass** der Anteil von Stearinsäure von 0,4 bis 2,2 Gew.-%, bezogen auf das Gesamtgewicht der Emulsion, beträgt.

7. Emulsion oder Verwendung nach einem der vorhergehenden Ansprüche **dadurch gekennzeichnet, dass** der Anteil von Hydroxypropylstärkephosphat von 0,2 Gew.-% bis 3,8 Gew.-%, bevorzugt von 0,3 Gew.-% bis 3,5 Gew.-% und insbesondere bevorzugt von 0,4 Gew.-% bis 3 Gew.-%, bezogen auf das Gesamtgewicht der Emulsion, beträgt.

8. Emulsion oder Verwendung nach einem der vorhergehenden Ansprüche **dadurch gekennzeichnet, dass** die Emulsion Glyceryl Stearate enthält, wobei der Gesamtanteil von Glyceryl Stearate von 0,1 Gew.-% bis 5,0 Gew.-%, bevorzugt 0,5 Gew.-% bis 3,2 Gew.-% und insbesondere bevorzugt von 1,0 Gew.-% bis 2,5 Gew.-%, beträgt, wobei sich die Angaben auf das Gesamtgewicht der Emulsion beziehen.

9. Emulsion oder Verwendung nach einem der vorhergehenden Ansprüche **dadurch gekennzeichnet, dass** die Emulsion Glyceryl Stearate Citrate enthält, wobei der Gesamtanteil von Glyceryl Stearate Citrate von 0,1 Gew.-% bis 5,0 Gew.-%, bevorzugt 0,5 Gew.-% bis 3,2 Gew.-% und insbesondere bevorzugt von 0,8 Gew.-% bis 2,5 Gew.-%, beträgt, wobei sich die Angaben auf das Gesamtgewicht der Emulsion beziehen.

10. Emulsion oder Verwendung nach einem der vorhergehenden Ansprüche **dadurch gekennzeichnet, dass** die O/W-Emulsion Polyglyceryl-3 Diisostearate enthält, wobei es vorteilhaft ist, wenn der Anteil von 0,1 Gew.-% bis 3,1 Gew.-%, bevorzugt 0,15 Gew.-% bis 1,5 Gew.-% und insbesondere bevorzugt von 0,2 Gew.-% bis 1,0 Gew.-%, beträgt, wobei sich die Angaben auf das Gesamtgewicht der Emulsion beziehen.

11. Emulsion oder Verwendung nach einem der vorhergehenden Ansprüche **dadurch gekennzeichnet, dass** der Anteil der Ölphase der Emulsion von mehr als 2 Gew.-% bis 30 Gew.-%, bevorzugt von mehr als 5 Gew.-% bis 20 Gew.-% und insbesondere bevorzugt von mehr als 8 Gew.-% bis 15,5 Gew.-%, bezogen auf das Gesamtgewicht der Emulsion, beträgt, wobei Tenside und Emulgatoren definitionsgemäß nicht zur Ölphase zählen.

12. Emulsion oder Verwendung nach einem der vorhergehenden Ansprüche **dadurch gekennzeichnet, dass** die Emulsion ein oder mehrere Fettalkohole mit 14 bis 22 Kohlenstoffatomen enthält, wobei der Gesamtanteil dieser Fettalkohole mit 14 bis 22 Kohlenstoffatomen vorteilhaft von 0,1 Gew.-% bis 8,0 Gew.-%, bevorzugt 0,5 Gew.-% bis 7,0 Gew.-% und insbesondere bevorzugt von 1 Gew.-% bis 6,0 Gew.-% beträgt, bezogen auf das Gesamtgewicht der Emulsion.

13. Emulsion oder Verwendung nach einem der vorhergehenden Ansprüche **dadurch gekennzeichnet, dass** die Emulsion weitere Tenside und/oder Emulgatoren nur in einem Maximalanteil von 2 Gew.-%, bevorzugt in einem Maximalanteil von 1,5 Gew.-% enthält, wobei sich die Angaben auf das Gesamtgewicht der Emulsion beziehen.

14. Emulsion oder Verwendung nach einem der vorhergehenden Ansprüche **dadurch gekennzeichnet, dass** neben Hydoxypropylstärkephosphat keine weiteren Stärken und Stärkederivate enthält.

## Claims

1. Acrylate- and silicone-free cosmetic O/W emulsion comprising
a) from 0.1 to 6% by weight of at least one fatty acid having 12 to 22 carbon atoms, and
b) from 0.1 to 5% by weight hydroxypropyl starch phosphate;
based in each case on the total weight of the emulsion, **characterized in that**
- the O/W emulsion does not comprise any mineral oil,
- palmitic acid and stearic acid are present, and
- in addition to palmitic acid and stearic acid, other fatty acids are present, the ratio by weight of the total amount of palmitic acid and stearic acid to the total amount of other fatty acids being from 8:1 to 99:1.

2. Use of a mixture of
a) at least one fatty acid having 12 to 22 carbon atoms, and
b) hydroxypropyl starch phosphate,
in an acrylate- and silicone-free cosmetic O/W emulsion for reducing white residues when the emulsion is applied to the skin, **characterized in that**
- the O/W emulsion does not comprise any mineral oil,
- palmitic acid and stearic acid are present, and
- in addition to palmitic acid and stearic acid, other fatty acids are present, the ratio by weight of the total amount of palmitic acid and stearic acid to the total amount of other fatty acids being from 8:1 to 99:1.

3. Emulsion according to Claim 1 or use according to Claim 2, **characterized in that** the fatty acids are selected from the group of palmitic acid, stearic acid, myristic acid, arachidic acid and oleic acid.

4. Emulsion or use according to any of the preceding claims, **characterized in that** the total fraction of fatty acids having 12 to 22 carbon atoms is from 0.2 to 5% by weight and preferably from 0.5 to 3% by weight, based in each case on the total weight the emulsion.

5. Emulsion or use according to any of the preceding claims, **characterized in that** the fraction of palmitic acid is from 0.4 to 2.2% by weight, based on the total weight of the emulsion.

6. Emulsion or use according to any of the preceding claims, **characterized in that** the fraction of stearic acid is from 0.4 to 2.2% by weight, based on the total weight of the emulsion.

7. Emulsion or use according to any of the preceding claims, **characterized in that** the fraction of hydroxypropyl starch phosphate is from 0.2% by weight to 3.8% by weight, preferably from 0.3% by weight to 3.5% by weight and particularly preferably from 0.4% by weight to 3% by weight, based on the total weight of the emulsion.

8. Emulsion or use according to any of the preceding claims, **characterized in that** the emulsion comprises glyceryl stearate, wherein the total fraction of glyceryl stearate is from 0.1% by weight to 5.0% by weight, preferably 0.5% by weight to 3.2% by weight and particularly preferably from 1.0% by weight to 2.5% by weight, the figures being based on the total weight of the emulsion.

9. Emulsion or use according to any of the preceding claims, **characterized in that** the emulsion comprises glyceryl stearate citrate, wherein the total fraction of glyceryl stearate citrate is from 0.1% by weight to 5.0% by weight, preferably 0.5% by weight to 3.2% by weight and particularly preferably from 0.8% by weight to 2.5% by weight, the figures being based on the total weight of the emulsion.

10. Emulsion or use according to any of the preceding claims, **characterized in that** the O/W emulsion comprises polyglyceryl-3 diisostearate, it being advantageous when the fraction is from 0.1% by weight to 3.1% by weight, preferably 0.15% by weight to 1.5% by weight and particularly preferably from 0.2% by weight to 1.0% by weight, the figures being based on the total weight of the emulsion.

11. Emulsion or use according to any of the preceding claims, **characterized in that** the fraction of the oil phase of the emulsion is from more than 2% by weight to 30% by weight, preferably from more than 5% by weight to 20% by weight and particularly preferably from more than 8% by weight to 15.5% by weight, based on the total weight of the emulsion, with surfactants and emulsifiers by definition not being included in the oil phase.

12. Emulsion or use according to any of the preceding claims, **characterized in that** the emulsion comprises one or more fatty alcohols having 14 to 22 carbon atoms, the total fraction of these fatty alcohols having 14 to 22 carbon atoms advantageously being from 0.1% by weight to 8.0% by weight, preferably 0.5% by weight to 7.0% by weight and particularly preferably from 1% by weight to 6.0% by weight, based on the total weight of the emulsion.

13. Emulsion or use according to any of the preceding claims, **characterized in that** the emulsion comprises further surfactants and/or emulsifiers only at a maximum fraction of 2% by weight, preferably at a maximum fraction of 1.5% by weight, the figures being based on the total weight of the emulsion.

14. Emulsion or use according to any of the preceding claims, **characterized in that** the emulsion does not comprise any other starches and starch derivatives apart from hydroxypropyl starch phosphate.

## Revendications

1. Émulsion H/E cosmétique, exempte d'acrylate et de silicone, contenant
a) 0,1 à 6% en poids d'au moins un acide gras présentant 12 à 22 atomes de carbone et
b) 0,1 à 5% en poids de phosphate d'hydroxypropylamidon ;
à chaque fois par rapport au poids total de l'émulsion, **caractérisée en ce que**
- l'émulsion HIE ne contient pas d'huile minérale,
- de l'acide palmitique et de l'acide stéarique sont contenus et
- d'autres acides gras, outre l'acide palmitique et l'acide stéarique, sont contenus, le rapport en poids de la proportion totale d'acide palmitique et d'acide stéarique par rapport à la proportion totale des autres acides gras étant de 8:1 à 99:1.

2. Utilisation d'un mélange constitué par
a) au moins un acide gras présentant 12 à 22 atomes de carbone et
b) du phosphate d'hydroxypropylamidon, dans une émulsion H/E cosmétique, exempte d'acrylate et de silicone, pour la réduction de résidus blancs lors de l'application de l'émulsion sur la peau, **caractérisée en ce que**
- l'émulsion HIE ne contient pas d'huile minérale,
- de l'acide palmitique et de l'acide stéarique sont contenus et
- d'autres acides gras, outre l'acide palmitique et l'acide stéarique, sont contenus, le rapport en poids de la proportion totale d'acide palmitique et d'acide stéarique par rapport à la proportion totale des autres acides gras étant de 8:1 à 99:1.

3. Émulsion selon la revendication 1 ou utilisation selon la revendication 2, **caractérisée en ce que** les acides gras sont choisis dans le groupe acide palmitique, acide stéarique, acide myristique, acide arachidonique ainsi qu'acide oléique.

4. Émulsion ou utilisation selon l'une des revendications précédentes, **caractérisée en ce que** la proportion totale d'acides gras présentant 12 à 22 atomes de carbone est de 0,2 à 5% en poids et de préférence de 0,5 à 3% en poids, à chaque fois par rapport au poids total de l'émulsion.

5. Émulsion ou utilisation selon l'une des revendications précédentes, **caractérisée en ce que** la proportion d'acide palmitique est de 0,4 à 2,2% en poids par rapport au poids total de l'émulsion.

6. Émulsion ou utilisation selon l'une des revendications précédentes, **caractérisée en ce que** la proportion d'acide stéarique est de 0,4 à 2,2% en poids par rapport au poids total de l'émulsion.

7. Émulsion ou utilisation selon l'une des revendications précédentes, **caractérisée en ce que** la proportion de phosphate d'hydroxypropylamidon est de 0,2% en poids à 3,8% en poids, de préférence de 0,3% en poids à 3,5% en poids et en particulier de préférence de 0,4% en poids à 3% en poids par rapport au poids total de l'émulsion.

8. Émulsion ou utilisation selon l'une des revendications précédentes, **caractérisée en ce que** l'émulsion contient du stéarate de glycéryle, la proportion totale de stéarate de glycéryle étant de 0,1% en poids à 5,0% en poids, de préférence de 0,5% en poids à 3,2% en poids et en particulier de préférence de 1,0% en poids à 2,5% en poids, les indications se rapportant au poids total de l'émulsion.

9. Émulsion ou utilisation selon l'une des revendications précédentes, **caractérisée en ce que** l'émulsion contient du stéarate-citrate de glycéryle, la proportion totale de stéarate-citrate de glycéryle étant de 0,1% en poids à 5,0% en poids, de préférence de 0,5% en poids à 3,2% en poids et en particulier de préférence de 0,8% en poids à 2,5% en poids, les indications se rapportant au poids total de l'émulsion.

10. Émulsion ou utilisation selon l'une des revendications précédentes, **caractérisée en ce que** l'émulsion HIE contient du diisostéarate de polyglycéryle-3, sa proportion étant avantageusement de 0,1% en poids à 3,1% en poids, de préférence de 0,15% en poids à 1,5% en poids et en particulier de préférence de 0,2% en poids à 1,0% en poids, les indications se rapportant au poids total de l'émulsion.

11. Émulsion ou utilisation selon l'une des revendications précédentes, **caractérisée en ce que** la proportion de la phase huileuse de l'émulsion est supérieure à 2% en poids à 30% en poids, de préférence supérieure à 5% en poids à 20% en poids et en particulier de préférence supérieure à 8% en poids à 15,5% en poids, par rapport au poids total de l'émulsion, les tensioactifs et les émulsifiants n'appartenant par définition pas à la phase huileuse.

12. Émulsion ou utilisation selon l'une des revendications précédentes, **caractérisée en ce que** l'émulsion contient un ou plusieurs alcools gras comprenant 14 à 22 atomes de carbone, la proportion totale de ces alcools gras comprenant 14 à 22 atomes de carbone étant avantageusement de 0,1% en poids à 8,0% en poids, de préférence de 0,5% en poids à 7,0% en poids et en particulier de préférence de 1% en poids à 6,0% en poids, par rapport au poids total de l'émulsion.

13. Émulsion ou utilisation selon l'une des revendications précédentes, **caractérisée en ce que** l'émulsion ne contient d'autres tensioactifs et/ou émulsifiants qu'en une proportion maximale de 2% en poids, de préférence en une proportion maximale de 1,5% en poids, les indications se rapportant au poids total de l'émulsion.

14. Émulsion ou utilisation selon l'une des revendications précédentes, **caractérisée en ce qu'**aucun autre amidon ni dérivé d'amidon n'est contenu en plus du phosphate d'hydroxypropylamidon.
